# EUROPEAN PATENT SPECIFICATION

(11) **EP 2 626 071 B1**
(45) Date of publication and mention of the grant of the patent: **13.01.2016**
(21) Application number: 11830238.9
(22) Date of filing: 08.10.2011
(51) Int. Cl.: A61K 31/365, A61K 9/107, A61P 7/00, A61P 7/06, A61P 43/00, A61K 47/10, A61K 47/14, A61K 47/44, A61K 9/50, A61K 9/48

(54) **ARCTIGENIN FOR USE IN PREVENTING OR TREATING BONE MARROW SUPPRESSION**
ARCTIGENIN ZUR ANWENDUNGEN BEI DER PROPHYLAXE ODER BEHANDLUNG VON MYELOSUPPRESSION
ARCTIGENINE POUR UTILISATION DANS LA PRÉVENTION OU LE TRAITEMENT DE LA SUPPRESSION DE LA MOELLE OSSEUSE

(30) Priority: 08.10.2010 CN 201010299279
(43) Date of publication of application: 14.08.2013
(73) Proprietor: Lunan Pharmaceutical Group Corporation, Linyi, Shandong 276006 (CN)
(72) Inventor: ZHAO, Zhiquan, Linyi Shandong 276006 (CN)
(74) Representative: Høiberg A/S
(86) International application number: PCT/CN2011/080543
(87) International publication number: WO 2012/045282

(56) References cited:
- CN-A- 101 036 643
- CN-A- 102 210 653
- DATABASE WPI Week 200531 Thomson Scientific, London, GB; AN 2005-296827 XP002720500, & CN 1 560 265 A (UNIV GUANGZHOU TRADITIONAL CHINESE MEDIC) 5 January 2005 (2005-01-05)

## Description

### Technical Field

The present invention relates to pharmaceutical uses of Arctigenin, especially to its use for preventing or treating diseases related to blood cell reduction, which belongs to the field of the therapeutic activity of the compound.

### Background

Normal blood contains a large number of cells, including red blood cells for transferring oxygen and white blood cells (also referred as leukocytes) for anti-infection. Leukocytes include neutrophils, eosinophils and basophils. Leukocytes are derived via bone marrow hematopoiesis. Normal blood also contains platelets. Platelets are tiny cell debris initiating blood coagulation. Blood cells of the human body are generated by the hematopoietic system. Human hematopoietic system consists of a small amount of bone marrow hematopoietic stem cells and different series of hematopoietic cells at different developmental stages, which is very sensitive to various physicochemical factors and harmful factors generated by *in vivo* metabolism, such as body fatigue, exposure to radiation or certain chemotherapy drugs, which leads to diseases like anemia and bone marrow suppression (also referred as myelosuppression) induced by blood cell reduction. In addition, there is also the congenital blood cells reduction, such as idiopathic thrombocytopenic purpura.

Cancer patients have to recieve radiotherapy and chemotherapy based treatments in quite a long time. Radiotherapy and chemotherapy are therapies for treating cancers by radiation or cytotoxic agents. However, radiotherapy and the vast majority of chemotherapy treatments are nonspecific, which are toxic to normal rapidly dividing cells. Large dose of radiation is also toxic to normal rapidly dividing cells. This often produces a variety of side effects in patients who are subjected to chemotherapy and radiotherapy. Although other normal tissues may also be adversely affected, bone marrow is particularly sensitive to such specific proliferation treatment as chemotherapy or radiotherapy. Bone marrow suppression, i.e., decline in production of blood cells in bone marrow, is the side effect of this class, and is characterized as decline in myeloproliferation, decline in blood cell counts, decline in leukocytes in peripheral blood, decline in neutrophils and/or thrombocytopenia, or even aplastic anemia, which causes serious harm to the patient's quality of life, and may even be life-threatening.

Clinically, patients who are subjected to radiotherapy, chemotherapy are vulnerable to injury with varying degrees of bone marrow suppression, noting as decline in leukocytes in peripheral blood, decline in neutrophils and/or thrombocytopenia. Peripheral blood neutrophils in human body accounts for about 50-70% of the total number of leukocytes, its increase and decline has a direct impact on the changes in the total number of leukocytes, i.e., as the number of neutrophils increases, the number of leukocytes increases; as the number of neutrophils decreases, the total number of leukocytes decreases. Correlation in both their number is in the sense of consistency, i.e. the significance of the increase or decrease in neutrophils is essentially consistent with the significance of the increase or decrease in the total number of leukocytes. Patients with bone marrow suppression are vulnerable to infection. Deficiency in neutrophilic leukocytes and platelet is the main causes of morbidity and mortality after cancer treatment, which also leads to the high cost of cancer treatment.

However, radiotherapy and chemotherapy are still the most commonly employed means in the treatment of tumors. Radiotherapy and chemotherapy-induced complications like bone marrow hematopoietic suppression has become an important factor that affects the quality of life in patients. Chemical drugs for auxiliary treatment of tumors exhibiting definite efficacy themselves will produce more adverse reactions after use, and making the patients even more painful. Many medical workers are committed to looking for some effective drugs with fewer side effects to against the damages induced by radiotherapy and chemotherapy.

The clinical measure currently adopted to bone marrow suppression is mostly to give a variety of growth factor in order to increase the proliferation of hematopoietic cells. The recombinant hematopoietic growth factors which have been most recently appeared on the market, such as Levcomax (rhGM-GSF), Filgrastim (rhG-CSF) have a significant effect in increasing the number of leukocytes, which, however, is too expensive to be afforded by most of the patients. Further, rhGM-GSF, rhG-CSF cannot be used concurrently with chemotherapy, and neither can it be used for prevention purpose, excepted that when reduction in leukocytes occurs to avoid the toxic effects to be generated.

In addition, the use of hematopoietic growth factors in gene therapy, such as IL-6, IL-3 gene therapy, is still in the animal testing stage. Further, autologous bone marrow transplantation is often used in conjunction with high-dose chemotherapy, which, however, is difficult to be applied repeatedly.

Therefore, development of a safe, effective, inexpensive method for preventing and treating blood cell reduction, particularly blood cell reduction induced by the side effects of chemotherapy and radiotherapy, as well as prompting the white blood cell counts after chemotherapy and radiotherapy, are significant for improving the effect of chemotherapy and radiotherapy against tumors, prolonging the survival period of cancer patients, improving the quality of life.

Burdock (Arctium *lappa*; Latin name, Arctium lappal) is a genus of *Arctium* plants of the Asteraceae family. Both Arctiin and Arctigenin are derived from the dried ripe fruit of burdock, namely Arctium lappa Linne. Due to the low activity of Arctiin, arctiin must be converted to Arctigenin via metabolism before being absorbed by the human body, whereas Arctigenin is an active ingredient which can be directly absorbed by human body, having the structure as shown below:

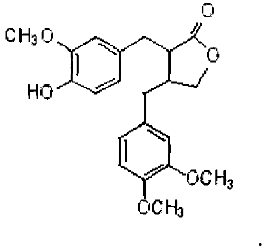

Currently, it has been reported in literature that Arctigenin has the following pharmacological activity:
1) anti-inflammatory and immunomodulatory effects;
2) antiviral activity, including HIV-1 and influenza virus;
3) induction of apoptosis of tumor cells;
4) therapeutic effect on nephropathy and diabetes, the complications of diabetes;
5) heat shock response inhibition;
6) neuroprotective effects;
7) dilation of blood vessels;
8) antagonism of platelet activating factor;
9) anti-Alzheimer's;
10) inhibition of K⁺ contracture and the like.

For example, Cho J Y (Cho J Y, et al., "Immunomodulatory effect of arctigenin, a lignan compound on tumor necrosis factor-α and nitric oxide production, and lymphocyte proliferation[J]". J Pharm Pharmcol. 1999; 51(11): 1267-1273) discloses that Arctigenin has anti-inflammatory and immunomodulatory effects. Gao Y (Gao Y, et al., "in vitro anti-influenza virus activity of Arctigenin", Chinese Traditional and Herbal Drugs, 2002, (8):724-726) discloses that Arctigenin has antiviral activity. Kim S H (Kim S H, et al., "Hepatoprotective dibenzylbutyrolactone lignans of Torreya nucifera against CCl4-induced toxicity in primary cultured rat hepatocytes[J]". Biol Pharm Bull. 2003; 26(8): 1202-1205) discloses that Arctigenin has the effect of inducing apoptosis of tumor cells. The above references are incorporated herein by reference.

In addition, the extraction process for separating Arctigenin has also been reported. For example, Zeng Y (Zeng Y, et al., "Lack of significant modifying effect of arctiin on prostate carcinogenesis in probasin/SV40 T antigen transgenic rats[J]". Cancer Lett. 2005; 222(2): 145-151) discloses a method of producing Arctigenin by hydrolysis of Arctiin using snailase. Also, <Handbook of extracting chemical components of Chinese traditional medicine> (Edited by Yang Y, et al., China Press of Traditional Chinese Medicine, Beijing; page 77) has reported a method of producing Arctigenin having a purity of ≥90% as determined by HPLC. Chinese patent application CN101134031A discloses two methods for producing Arctigenin and the method of preparing tablets, soft capsules, pills, and injection thereof. The above references are incorporated herein by reference.

Chinese patent application CN1560265A discloses methods for preparing arctigenin for treating various diseases e.g. leukemia.

Chinese patent application CN 101036643A discloses pharmaceutical compositions comprising arctigenin and capable of producing emulsions.

There is no literature reporting the activity of Arctigenin in preventing or treating blood cell reduction, particularly the activity in preventing or treating radiation or chemical induced bone marrow suppression.

### Detailed description

The present invention is as defined in the claims.

For the shortcomings of the existing drugs used in preventing or treating blood cell reduction related diseases, particularly for preventing and treating bone marrow suppression, an objective of the present invention is to provide a drug for preventing and/or treating blood cell reduction, particularly for preventing or treating radiation or chemical induced bone marrow suppression.

The inventor has long been committed to studying the anticancer activity of Arctigenin with a number of achievements. Recently, the inventor unexpectedly found the unexpected activity of Arctigenin in enhancing immunity and promoting hematopoietic function during the process of further in-depth study.

According to the above findings, the objective of the present invention is achieved. More specifically, the present invention comprises the following technical solutions:
The first aspect of the present invention is to provide Arctigenin for use in preventing or treating bone marrow suppression induced by radiation or a chemical.

"Arctigenin" refers to a compound having the aforementioned structure, which may be obtained from extraction, biological synthesis, or chemical synthesis, including Arctigenin, pharmaceutically acceptable salts or possible hydrates thereof. The Arctigenin in the present invention refers to biological or chemical products, of which the purity is ≥90%, ≥95% or ≥98% as determined by HPLC.

Any methods for preparing Arctigenin which has been disclosed prior to the application date of the present application can be used as the method for preparing arctigenin of the present invention

For example, the preparation can be achieved according to the method as described by Zeng Y, et. al. (see texts above) or the method as disclosed in the Chinese patent application CN101134031A. For example, extraction of Arctigenin may comprise the following steps:
(1) Taking the crude powder of burdock, adding an appropriate amount of acid to adjust the pH to acidic for hydrolysis to take place;
(2) Discarding the hydrolyzate, washing the aqueous solution of burdock with water until the pH reaches neutral, drying to obtain product A;
(3) Dissolving product A obtained from (2);
(4) Cool-precipitating the resultant solution obtained from (3), and removing the precipitate (e.g. by centrifugal machine);
(5) Obtaining product B from the supernatant of (4);
(6) Extracting product B to obtain crude Arctigenin extract (60%~70% Arctigenin);
(7) Purifying the crude Arctigenin extract (e.g. purified by preparative high performance liquid chromatography) to give Arctigenin of high purity (≥99.3%).

Blood cell reduction can be induced by a variety of causes. For example, it may be the idiopathic or congenital one, or it may be the radiation or chemical induced blood cell reduction, including various reductions of blood cell, such as erythropenia, thrombocytopenia, various leukopenia and the like. Diseases related to blood cell reduction refer to diseases induced, mediated or caused by blood cell reduction, including, for example, thrombocytopenia, bone marrow suppression, anemia, and other idiopathic or secondary blood diseases related to blood cell reduction, which, however, are not limited thereto.

In the present invention, said "thrombocytopenia" refers to a condition characterized by platelet defects, and includes thrombocytopenia induced by radiotherapy or chemotherapy, like anti-cancer drugs, and congenital thrombocytopenia, such as idiopathic thrombocytopenic purpura (ITP), etc. Currently, platelet loss induced by anti-cancer drugs is the rate-limiting step in chemotherapy. For patients with severe thrombocytopenia, treatment must be reduced or suspended, until the platelet level is elevated to a level higher than the threshold value. Clinical symptoms found in ITP patients are identical to that in patients with chemotherapy-induced thrombocytopenia, which include increased risk of bleeding, and even life-threatening bleeding, etc.

In the present invention, said "bone marrow suppression" or "myelosuppression" refers to separate or simultaneous reduction of blood cells, including leukocytes, erythrocytes, neutrophils or platelets, and is characterized as decline in production of blood cell or platelet. Healthy bone marrow generates every day a large number of red blood cells, leukocytes and platelet. Under the bone marrow suppression, these cells produced by the bone marrow decline. One characteristic of bone marrow suppression is decline in production of leukocytes. This decline in production of leukocytes may be induced by certain treatments, particularly cancer treatments, for example, chemotherapy and radiotherapy. Preferably, said bone marrow suppression in the present invention is characterized as decline in the number of peripheral blood leukocytes or thrombocytopenia, bone marrow dysplasia or aplastic anemia. In one embodiment, said peripheral leucopenia refers to reduction in peripheral blood neutrophils.

In the present invention, said "anemia" refers to the condition wherein the number of erythrocytes and hemoglobin in blood, or the packed cell volume are decreased below the normal value, according to the characterization by the hematocrit determination. Anemia may be classified, according to causes to anemia, for example, into aplastic anemia, hemolytic anemia, iron deficiency anemia and sickle cell anemia, which, however, is not limited thereto. Among these, aplastic anemia is characterized as lacking of red blood cell regeneration and resistance to treatment. In these patients, the myeloid cells, erythrocyte series and thrombopoietic stem cells significantly decrease, which leads to pancytopenia.

It can be seen that the basis pathology and clinical symptoms of the above diseases exhibit as blood cell reduction, which are consistent among the above diseases. In this regard, based on the activity of Arctigenin of the present invention in enhancing immunity and promoting hematopoiesis, the present invention provides Arctigenin for use in preventing or treating diseases related to blood cell reduction.

Said blood cell reduction is preferably thrombocytopenia and leukopenia, particularly neutropenia. Among the above-mentioned diseases, the present invention is that Arctigenin is preferably used for preventing or treating bone marrow suppression, more preferably radiation or chemical induced bone marrow suppression.

Accordingly, in one specific embodiment, the present disclosure provides the use of Arctigenin in medicaments for preventing or treating radiation or chemical induced bone marrow suppression.

Said "radiation" may be the radiation received during the process of radiotherapy, or those radiations of other sources, such as those of working or environmental causes. Preferably, the radiations according to the use of the present invention are received in the process of radiotherapy, for example, radiations received during the radiotherapy process for the treatment of cancers.

Said "chemicals" may refer to any drugs or chemicals capable of causing the blood cell reduction. In one embodiment, the chemicals according to the use of the present invention are chemotherapeutic drugs for treating cancer. Said chemotherapeutic drugs include alkylating drugs, anti-metabolic drugs, antibiotics, plant-based drugs and hormone drugs, which, however, are not limited thereto. The alkylating drugs include, but not limited to, mechlorethamine, cyclophosphamide, thiotepa, cyclohexylnitrosourea, Myleran, dacarbazine and methylbenzyl hydrazine; said anti-metabolic drugs include, but not limited to, fluorouracil (5-FU), ftorafur (FT-207), Tegadifur (FD-1), uracil-tegafur (UFT), furtulon (5-DFUR), methotrexate (MTX), aminopterinum, arabinocytidine (Ara-c), cyclocytidine, cyclocylidine hydrochloride, hydroxyurea (HU), inosine dialdehyde, adenosine dialdehyde (adenosinediialde-hgde), guanazole, 6-mercaptopurine (6-MP); said antibiotics includes, but not limited to, penicillins antibiotic, cephalosporins antibiotic, aminoglycosides antibiotics, macrolides antibiotic, sulfonamides antibiotic, quinolones antibiotics, furans antibiotic; said plant-based drugs are plant-derived drugs, which include traditional Chinese herbal prescription drugs and those obtained from the effective parts of plants by modern means of extraction and separation or Chinese medicine monomer drugs; said hormone drugs include, glucocorticoid, adrenal cortical hormone, norepinephrine hormone, progesterone, estrogen, androgen.

Preferably, said chemotherapy drugs for treating cancer are Docetaxel, imatinib mesylate, or a combination thereof.

The second aspect of the present invention relates to a method of preventing or treating diseases related to blood cell reduction, which comprises the step of administering to the subjects in need of said treatment or prevention an effective amount of Arctigenin.

Said "subjects" may be mammals, such as human.

Said "effective amount" includes a therapeutically effective amount and a prophylactically effective amount. "A therapeutically effective amount" refers to an amount capable of achieving the desired therapeutic results (e.g. the desired result of treatment of bone marrow suppression) as administered within a necessary period of time. It is understood that a therapeutically effective amount of arctigenin varies according to the route of administration, different nature of the treatment, the age, sex, weight, and health status of patients, as well as the type of radiotherapy or chemotherapy and other factors, which is ultimately decided by the clinicians involved in the treatment. The dosing regimen may be adjusted to provide optimal treatment effects. A therapeutically effective amount can also be an amount of which the beneficial therapeutic effect more than any toxic or harmful effects. "A prophylactically effective amount" refers to an amount capable of achieving the desired preventive effect (e.g., prevention or protection of bone marrow function, or the maintenance of the hematopoietic cells) as administered within a necessary period of time. The prophylactic administration is usually taken ahead of the occurrence of diseases or at the earlier stage of diseases. Accordingly, a prophylactically effective amount may be lower than a therapeutically effective amount.

An effective amount of Arctigenin used for preventing or treating blood cell reduction, preferably for preventing or treating bone marrow suppression, particularly the radiation or chemical induced bone marrow suppression ranges from 0.1 to 1000 mg/kg/d, preferably 1~100 mg/kg/d, more preferably 5 to 50 mg/kg/d, for example, 10 mg/kg/d.

In addition, according to the present invention,

Arctigenin can be used as the sole active ingredient for preventing and/or treating diseases related to blood cell reduction, preferably for preventing or treating bone marrow suppression, particularly the radiation or chemical induced bone marrow suppression, and may also be used in combination with other drugs for achieving such prevention and/or treatment.

Other drugs that may be used in combination with Arctigenin may be one or more of other drugs for use in treating leukopenia (such as vitamin B4, leucogen, batilol, coenzyme A), or one or more drugs capable of increasing the number of platelet (such as interleukin-II, low-dose glucocorticoids, such as prednisone) for preventing or treating blood cell reduction, particularly for preventing or treating bone marrow suppression.

According to one embodiment, as used in combination with other drugs in the medical use of the present invention, the weight ratio of two drugs can be appropriately adjusted according to the condition and symptoms of the patients, the weight ratio of other drugs and Arctigenin preferably ranges from (0.005-100): 1, more preferably (0.005-50): 1, most preferably (0.01-100): 1, for example (0.05-25): 1.

Although Arctigenin can be used in combination with other drugs, Arctigenin is preferably used as the sole active ingredient for preventing and/or treating radiation or chemical induced bone marrow suppression.

The administration routes of Arctigenin and optionally other drugs include gastrointestinal route and parenteral route. Parenteral route includes subcutaneous, intradermal, arterial, intravenous, intramuscular, articular, intrathecal, intracranial, intrapleural, intraperitoneal injection or infusion, nasal, buccal, sublingual, intratracheal, intraurethral, intrarectal, or local administration.

In the use of arctigenin in preventing and/or treating radiation or chemical induced bone marrow suppression, Arctigenin may be administered before, during or after radiotherapy or chemotherapy. In the combined use of Arctigenin with other drugs, Arctigenin may be administered concurrently or sequentially with other drugs.

In a further aspect the present invention relates to a formulation for preventing or treating diseases related to blood cell reduction, which comprises Arctigenin and pharmaceutically acceptable pharmaceutical excipients. Said formulation is preferably used for preventing or treating bone marrow suppression, particularly the radiation or chemical induced bone marrow suppression.

In the present invention, "pharmaceutically acceptable pharmaceutical excipients" refer to any substances that do not interfere with the physiological role of Arctigenin, and being non-toxic to subjects including humans.

The pharmaceutical excipients used in the formulation of the present invention are conventional excipients well-known in the art. Appropriate pharmaceutical excipients are described in details in <The Complete Collection of Pharmaceutical Excipients> (Page 123, Sichuan Technology and Science Press, 1993, Edited by LUO Ming-sheng, GAO Tian-hui). For example, the pharmaceutical excipients commonly employed for preparing microemulsion formulation include, but not limited to, soybean oil, polyoxyethylene-23-lauryl ether, 1,2-propanediol, hydrogenated coconut oil glycerides, lauroyl polyethylene glycol-32-glyceride, polyethylene glycol 3350, safflower oil, cotton seed oil, decaglyceryl monostearate; the pharmaceutical excipients commonly employed for preparing dropping pill formulation include, but not limited to, polyethylene glycol 6000, polyethylene glycol 1000; the pharmaceutical excipients commonly employed for preparing capsule formulation include lactose and corn starch. Pharmaceutically acceptable carriers commonly employed for preparing soft capsule formulation include medium chain fatty acid glyceride, polyoxyethylene castor oil and 1,2-propanediol.

Pharmaceutical excipients can be suitably selected by a person skilled in the art according to the actual needs, by which, the formulation of the present invention can be formulated according to the method commonly employed in the art (see, for example, the Chinese patent application CN101134031A). Said formulation includes solids, liquids, oils, emulsions, gels, aerosols, inhalation, spray, capsule, pills, patches and suppositories.

According to one preferred embodiment, the dosage forms of the formulation of the present invention are those adapted to the gastrointestinal or parenteral administration, preferably tablets, emulsions, microemulsion formulation, capsule formulation, dropping pill or soft capsule, more preferably microemulsion formulation having the preferable mean particle diameter of 15-80 nm.

The beneficial effects of Arctigenin in preventing or treating diseases related to blood cell reduction, particularly in preventing or treating radiation or chemical induced bone marrow suppression include:
1) prevention of the decline in leukocytes in the treatment of cancer, and even the number of neutrophils, whereby enhancing the anti-tumor chemotherapy effect, prolonging the survival period of cancer patients, improving the quality of life. From the experimental effects of the effect of Arctigenin on the bone marrow suppression induced by chemotherapy in tumor-bearing mice, it is found that Arctigenin has the unexpected effect on alleviating bone marrow suppression. More specifically, the Arctigenin group showed an increase in both the total white blood cell count and the total neutrophil count with an extremely significant difference (P<0.01) or significant difference (P<0.05) as comparing with the model group; meanwhile, the Arctigenin group showed significant increases in total white blood cell count and total neutrophil count with an extremely significant difference (P < 0.01) as comparing with the Docetaxel group and the imatinib mesylate group.
2) Arctigenin can be used simultaneously with chemotherapeutic agents. Arctigenin can also be used for prophylaxis rather than administered only after the appearance of leucopenia, so as to reduce side effects to some extent Additionally, it can be seen from the experimental results that, Arctigenin, even used as an anti-tumor agent in combination with Docetaxel or Imatinib Mesylate, not only functions to inhibit tumor growth, but also relieves myelosuppression caused by Docetaxel or Imatinib Mesylate. Therefore, Arctigenin can be used simultaneously with chemotherapeutic agents. Arctigenin can also be used for prophylaxis rather than administered only after the appearance of leucopenia, so as to insure the tumor-inhibiting rate and promote bone marrow hematopoiesis at the same time, increase white blood cell count and neutrophil count, and reduce side effects to some extent. The effects of Arctigenin for relieving myelosuppression may relate with its ability for promoting the proliferation and re-generation of hemopoietic stem cells in bone marrow. As can be seen from the experimental data in Table 1, there is no significant difference in the weight of mice in Arctigenin groups (either administered alone or in combination with other agents) with mice in Model Group during the period of administration. No significant difference was observed in the feed intake and living status of the animals. The results indicate that Arctigenin, as an anti-tumor agent and myelosuppression-releasing agent, does not have significant toxic or side effects.
3) Arctigenin has a significant effect for preventing or treating bone marrow suppression produced during the radiotherapy process. As can be seen from the experimental results of the effects of Arctigenin on white blood cell and platelet of mice received ⁶⁰Co radiation at 4Gy (Table 3), Arctigenin exhibited unexpected effects in alleviating myelosuppression caused by radiotherapy. Specifically, the Arctigenin group shows an increase in both total white blood cell count and the platelet count with extremely significant difference (P < 0.01) or significant difference (P < 0.05) in comparison with the model group; and also a significant difference (*P* < 0.05) as comparing with burdock extract group.

### Examples

### Example 1 Preparation of Arctigenin

(1) taking 1 kg burdock, adding an appropriate amount of hydrochloric acid or sulphuric acid to adjust the pH to about 1.5 for undergoing hydrolysis at 65°C for 6 hours;
(2) discarding the acidic hydrolyzate, washing the aqueous solution of burdock extract with water until the pH reaches neutral, drying at 80°C to obtain product A;
(3) dissolving product A using an appropriate amount of ethanol, and heating to reflux to obtain the ethanol extract;
(4) cool-precipitating the resultant ethanol extract at 4°C, and removing the precipitate by centrifuging in a tube centrifuge at 2000r/min, collecting the supernatant from the centrifugation;
(5) drying the supernatant to obtain product B;
(6) grinding product B into powder, adding dichloromethane, extracting for 2.5 hours, distilling dichloromethane extract under reduced pressure to obtain the crude Arctigenin having the purity 65%;
(7) Purifying the crude Arctigenin extract by preparative high performance liquid chromatography to give the crude Arctigenin:
   The crude Arctigenin extract was dissolved in acetonitrile-water solvent (acetonitrile: water = 2:3, v/v), and purified by preparative high performance liquid chromatography after filtration. Octadecylsilane bonded silica microspheres (Sepax, purchased from Hai Shu Heng Long Shi Yan Qi Cai Co., Ltd., Ning Bo) having a particle size of 10um was used as the filler; mobile phase is composed of acetonitrile-water (acetonitrile: water = 2:3, v/v); flow rate: 260 ml/min to 330 ml/min, injection volume: 50-100ml; isocratic elution; UV detector online detection, the detection wavelength: 280nm. The preparative components of Arctigenin were specifically collected to afford the Arctigenin solution; the Arctigenin solution was subjected to distillation under reduced pressure to remove acetonitrile and water, giving Arctigenin of high purity (99.5% as determined by HPLC).

### Example 2: Arctigenin microemulsion formulation

| | |
|---|---|
| Arctigenin | 10g |
| soybean oil | 35g |
| polyoxyethylene-23-lauryl ether | 60g |
| 1,2-propanediol | 30g |

Preparation process: prescriptive amounts of soybean oil, polyoxyethylene-23-lauryl ether and 1,2-propanediol were weighed, mixed and stirred well. Arctigenin was added and dissolved, where ultrasonic treatment was optionally used for accelerating the dissolution, to give a clear solution, namely Arctigenin microemulsion formulation. Laser particle size analyzer was used to determine the particle size, which was determined as 15nm on average.

### Example 3: Arctigenin microemulsion formulation

| | |
|---|---|
| Arctigenin | 0.1g |
| hydrogenated coconut oil glycerides | 5g |
| lauroyl polyethylene glycol-32-glyceride | 20g |
| 1,2-propanediol | 5g |
| polyethylene glycol 3350 | 20g |

Preparation process: prescriptive amounts of hydrogenated coconut oil glycerides, lauroyl polyethylene glycol-32-glyceride, 1,2-propanediol and polyethylene glycol 3350 were weighed, mixed and stirred well, Arctigenin was added and dissolved, where ultrasonic treatment was optionally used for accelerating the dissolution, to give a clear solution, namely Arctigenin microemulsion formulation. Laser particle size analyzer was used to determine the particle size, which was determined as 40nm on average.

### Example 4: Arctigenin microemulsion formulation

| | |
|---|---|
| Arctigenin | 10g |
| safflower oil | 35g |
| polyoxyethylene-23-lauryl ether | 60g |
| 1,2-propanediol | 30g |

Preparation process: prescriptive amounts of safflower oil, polyoxyethylene-23-lauryl ether and 1,2-propanediol were weighed, mixed and stirred well. Arctigenin was added and dissolved, where ultrasonic treatment was optionally used for accelerating the dissolution, to give a clear solution, namely Arctigenin microemulsion formulation. Laser particle size analyzer was used to determine the particle size, which was determined as 36nm on average.

### Example 5: Arctigenin microemulsion formulation

| | |
|---|---|
| Arctigenin | 10g |
| Cotton seed oil | 30g |
| decaglyceryl monostearate | 55g |
| 1,2-propanediol | 25g |

Preparation process: prescriptive amounts of cotton seed oil, decaglyceryl monostearate and 1,2-propanediol were weighed, mixed and stirred well. Arctigenin was added and dissolved, where ultrasonic treatment was optionally used for accelerating the dissolution, to give a clear solution, namely Arctigenin microemulsion formulation. Laser particle size analyzer was used to determine the particle size, which was determined as 80nm on average.

### Example 6: Arctigenin microemulsion formulation

| | |
|---|---|
| Arctigenin | 2g |
| soybean oil | 30g |
| decaglyceryl monostearate | 55g |
| 1,2-propanediol | 25g |

Preparation process: prescriptive amounts of soybean oil, decaglyceryl monostearate and 1,2-propanediol were weighed, mixed and stirred well. Arctigenin was added and dissolved, where ultrasonic treatment was optionally used for accelerating the dissolution, to give a clear solution, namely Arctigenin microemulsion formulation. Laser particle size analyzer was used to determine the particle size, which was determined as 47nm on average.

### Example 7: Arctigenin microemulsion formulation

| | |
|---|---|
| Arctigenin | 1g |
| soybean oil | 30g |
| decaglyceryl monostearate | 55g |
| 1,2-propanediol | 25g |

Preparation process: prescriptive amounts of soybean oil, decaglyceryl monostearate and 1,2-propanediol were weighed, mixed and stirred well. Arctigenin was added and dissolved, where ultrasonic treatment was optionally used for accelerating the dissolution, to give a clear solution, namely Arctigenin microemulsion formulation. Laser particle size analyzer was used to determine the particle size, which was determined as 58nm on average.

### Example 8: Arctigenin dropping pills formulation

| | |
|---|---|
| Arctigenin | 5.0 g |
| polyethylene glycol -6000 | 14.5 g |
| polyethylene glycol -1000 | 5.0 g |
| | Formulated into 1000 pills |

Preparation process: a prescriptive amount of Arctigenin (sieved by a 100 mesh) was weighed, and added into a mixed solution comprising prescriptive amounts of polyethylene glycol 6000 and polyethylene glycol 1000 being melted on a heated water bath, mixed well, and placed in a dropping bottle, the dropping process was performed at of 95±2°C; dipping into a glass condenser column containing 4-6 mL of methyl silicone oil, which was then taken out after molding, and wiped off the methyl silicone oil attached thereon using an absorption paper.

### Example 9: enteric soft capsule formulation of Arctigenin

### Formulation of content material:

| | |
|---|---|
| Arctigenin | 10 g |
| anhydrous ethanol | 10 g |
| 1,2-propanediol | 10 g |
| polyoxyethylene castor oil | 50 g |
| medium chain fatty acid glycerides | 20 g |

### Formulation of capsule shell:

| | |
|---|---|
| gelatin | 10 g |
| glycerin | 5 g |
| purified water | 10 g |

### Formulation of entric coating solution:

| | |
|---|---|
| Eudragit L30D-55 | 100 g |
| triethyl citrate | 3 g |
| talc powder | 7.5 g |
| purified water | 200 g |

Preparation process: prescriptive amounts of medium chain fatty acid glycerides, polyoxyethylene castor oil, 1,2-propanediol and anhydrous ethanol were weighed, mixed and stirred well. Arctigenin was added and dissolved, where ultrasonic treatment was optionally used for accelerating the dissolution, to give a clear concentrated liquor, namely Arctigenin microemulsion concentrate. To the resultant microemulsion concentrate was added water according to a weight ratio of 1: 10-20 for dilution to give a clear solution, namely the soft capsule microemulsion fill material. Prescriptive amounts of gelatin, glycerin and purified water were weighed, mixed well and pressed into the capsule shell. Prescriptive amounts of Eudragit L30D-55, triethyl citrate, talc powder and purified water were weighed and mixed well to give the entric coating solution. The Arctigenin-containing soft capsule microemulsion content material was encapsulated in capsule shells and formulated into soft capsules, on which were coated with entric coating to provide the enteric soft capsules.

### Example 10: Arctigenin capsules

| | |
|---|---|
| Arctigenin | 100 g |
| lactose | 120 g |
| corn starch | 130 g |
| magnesium stearate | 5 g |

Preparation process: 100g of Arctigenin, 120g of lactose and 130g of corn starch were mixed in a mixer for 10-15 minutes, followed by addition of 5g of magnesium stearate, mixed for further 1-3 minutes, which was then encapsulated in 1000 capsule shells.

### Example 11 Effects of Arctigenin on Myelosuppression caused by chemotherapy in tumor-bearing mice

### 1. Materials

1.1 Animals: Kunming mice (purchased from National Institute for the Control of Pharmaceutical and Biological Products, China; Laboratory Animal NO: SCXK II - 00- 0010; female/male ratio at 1:1; Age: 7 weeks; Body weight: 18~22 g) were kept at 20 ± 1°C, 40%-70% humidity and offered with normal feed and water *ad libitum.*

### 1.2 Agents

| Agents | Sources/Manufacturers |
|---|---|
| Arctigenin | Prepared according to Example 1; Purity 99.3% |
| Docetaxel | Hangzhou Sanofi-Aventis Minsheng Pharmaceutical Co., Ltd. |
| Imatinib Mesylate | Beijing Novartis Pharma Ltd |
| Murine cancer cell line s180 | Shanghai Aiyan Biotechnical Co., Ltd. |
| 1640 culture medium | Shanghai Yansheng Biochemical Agents Co., Ltd. |

### 2. Methods

The murine cancer cell line s180 was cultured in 1640 culture medium at 37°C, 5% CO₂, sub-cultured every two days on average till logarithmic growth phase and then prepared into single cell suspension at 3.0 x 10⁷ cell/ml with physiological saline. The cell suspension was injected into abdominal cavity of mice under sterile conditions. The mice were sacrificed by cervical dislocation at 7 days after inoculation when the abdominal cavity expanded obviously, and then soaked in a flask with 75% ethanol for 2-3 min. The sterilized mice were placed on a superclean bench with abdomen exposed. The ascites were taken with sterile injectors and stored in sterile vials for further use. The ascites was counted by Trypan blue staining, and then diluted to 2.0 x 10⁷ cell/ml with physiological saline. The animals were inoculated with the dilutions in the right armpit at 0.2ml/animal.

The inoculated animals were divided randomly into following six groups with 10 animals each group (F:M = 1:1):
1. Model Group (Group M): receiving peritoneal injection of 0.9% physiological saline;
2. Docetaxel Group (Group D): receiving 75 mg/m²/d Docetaxel;
3. Imatinib Mesylate Group (Group IM): receiving 222.2 mg/m²/d Imatinib Mesylate;
4. Arctigenin Group (Group A): receiving 10 mg/m²/d Arctigenin;
5. Docetaxel plus Arctigenin Group (Group D + A): receiving 75 mg/m²/d Docetaxel plus 10 mg/m²/d Arctigenin;
6. Imatinib Mesylate plus Arctigenin Group (Group IM + A): receiving 222.2 mg/m²/d Imatinib Mesylate plus 10 mg/m²/d Arctigenin.

Docetaxel and Imatinib Mesylate were administered via tail vein injection at dosages of 20 ml/kg. Arctigenin was administered via peritoneal injection at a dosage of 10 ml/kg. The administration was given to each group once per day for 10 days in total. During the experimental process, the animals were observed everyday for their diets, survivals and behaviors and weighted every day. At the end of the experiment, the animals were anaesthetized and anatomize to draw blood from abdominal vena cava for blood routine examination, including total white blood cell count, total platelet count and neutrophil count.

### 3. Results

**Table 1: Effects of Arctigenin on Body Weight of mice**

| Groups | Sample number | Before administration (g) | the fifth day of administration (g) | The Tenth Day of administration (g) |
|---|---|---|---|---|
| Group M | 10 | 21.2 ± 2.6 | 30.3 ± 4.1 | 30.3 ± 4.3 |
| Group D | 10 | 21.2 ± 1.8 | 28.1 ± 2.5 | 27.2 ± 3.2 |
| Group IM | 10 | 21.3 ± 2.3 | 29.6 ± 3.2 | 30.0 ± 3.6 |
| Group A | 10 | 21.1 ± 3.0 | 27.9 ± 4.2 | 27.9 ± 3.8 |
| Group D+A | 10 | 21.0 ± 2.9 | 28.4 ± 3.7 | 27.3 ± 3.5 |
| Group IM+A | 10 | 21.3 ± 3.5 | 28.8 ± 3.0 | 30.1 ± 4.9 |

As can be seen from the experimental data in Table 1, there is no significant difference in the weight of mice between Arctigenin groups (either administered Arctigenin alone or in combination with other agents) and mice in Model Group during the period of administration. No significant difference was observed in the feed intake and living quality of the animals. The results indicate that Arctigenin, as an anti-tumor agent and myelosuppression-releasing agent, does not have significant toxic or side effects.

**Table 2: Effects of Arctigenin on neutrophil among white blood cells of Tumor-bearing mice**

| Groups | Sample number | White Blood Cells (10⁹/L) | Neutrophil (10⁹/L) | platelet (10⁹/L) |
|---|---|---|---|---|
| Group M | 10 | 2.831 ± 0.32 | 1.128 ±0.18 | 508.3 ± 38.6 |
| Group D | 10 | 2.773 ± 0.46 | 0.670 ±0.12 | 450.6 ± 30.5 |
| Group IM | 10 | 2.703 ± 0.37 | 0.695 ± 0.09 | 432.9 ± 34.8 |
| Group A | 10 | 3.530 ± 0.31^{##**$$} | 1.316 ± 0.15^{#**$$} | 687.4 ± 37.3^{##} |
| Group D+A | 10 | 3.288 ± 0.34^{#*$$} | 1.301 ± 0.16^{#**$$} | 598.7 ± 25.8^{#**} |
| Group IM+A | 10 | 2.895 ± 0.43 | 1.076 ±0.20^{**$$} | 623.1 ± 27.9^{##$$} |

| | | | | |
|---|---|---|---|---|
| Compared with Group M: ^{#}P < 0.05, ^{##}P < 0.01; Compared with Group D: *P < 0.05, **P < 0.01; Compared with Group IM: ^{$}P < 0.05, ^{$$}P < 0.01. | | | | |

As can be seen from the experimental effects of Arctigenin on myelosuppression caused by chemotherapy in tumor-bearing mice (Table 2), the neutrophil numbers of the mice in Group D or IM decreased significantly in comparison with Group M. In contrast, Arctigenin exhibited the unexpected effect on alleviating bone marrow suppression. More specifically, the Arctigenin group showed an increase in both the total white blood cell count and the total neutrophil count with extremely significant difference (*P*<0.01) or significant difference (*P*<0.05) as comparing with the model group; meanwhile, the Arctigenin group showed significant increases in total white blood cell count and total neutrophil count with an extremely significant difference (*P* < 0.01) as comparing with the Docetaxel group and the imatinib mesylate group.

Additionally, it can be seen from the experimental results that, Arctigenin, when used as an anti-tumor agent in combination with Docetaxel or Imatinib Mesylate, not only functions to inhibit tumor growth, but also relieve myelosuppression caused by Docetaxel or Imatinib Mesylate. Therefore, Arctigenin can be used simultaneously with other chemotherapeutic agents. Arctigenin can also be used for prophylaxis rather than administered only after the appearance of leucopenia, so as to insure the tumor-inhibiting rate and promote bone marrow hematopoiesis at the same time, increase white blood cell count and neutrophil count, and reduce side effects to some extent. The effects of Arctigenin for relieving myelosuppression may relate with its ability for promoting the proliferation and re-generation of hemopoietic stem cells in bone marrow.

### Example 12: Effects of Arctigenin on Blood cell counts of mice under ⁶⁰Co radiation

### 1. Materials

1.1 Animals: Kunming mice (purchased from National Institute for the Control of Pharmaceutical and Biological Products, China; Laboratory Animal NO: SCXK II - 00- 0010; female/male ratio at 1:1; Age: 7 weeks; Body weight: 18~22 g) were kept at 20 ± 1°C, 40%-70% humidity and offered with normal feed and water *ad libitum.*

### 1.2 Agents

| Agents | Sources/Manufacturers |
|---|---|
| Arctigenin | Prepared according to Example 1; Purity 99.3% |
| Extracts of *Arctium lappa* | Prepared according to the following extraction process |

### 2. Methods

Excepting the normal group (n = 10), the mice of the other groups all received ⁶⁰Co radiation at onetime of 4Gy (whole body radiation; absorbed dose = 4Gy; absorbed dose rate = 0.88Gy/min). At 3, 7 and 10 days after radiation, blood samples were collected from orbital vein for detection of whole blood cell count. The mice whose white blood cell counts were lower than 3.0 x 10⁹ /L or platelet counts were lower than 500 x 10⁹ /L in successive two whole blood cell detections were eliminated from the experiment. The rest mice were included in the experiment.

The mice fulfilled the experimental requirements after radiation were divided randomly into the following groups of: model group, group of burdock extract and Arctigenin group (each group: n = 10; F/M = 1:1). Each group was subjected to treatment or administration as follows:
1. Normal Group (Group N): peritoneal injection of 0.9% physiological saline at a volume of 10 ml/kg;
2. Model Group (Group M): peritoneal injection of 0.9% physiological saline at a dose of 10 mg/kg;
3. Group of burdock Extract (Group E): peritoneal injection of water exacts of *Arctium lappa* at a dose of 3.5 ml/kg/d;
4. Arctigenin Group (Group A): peritoneal injection of 10 mg/kg/d Arctigenin at a volume of 10 ml/kg.

Said burdock extract was prepared by: 1) placing burdock in a pot, adding water till the plant was immerged; 2) soaking burdock for 30min for promoting the extraction of the effective components; 3) rapidly heating till boiling, fully boiling for 1-3min, further heating for 20-30min for concentration, and filtrating through sterile gauzes into a beaker; and 4) mixing the first dose with re-extracted agents uniformly after the first extraction for balancing their effects. 200ml water extracts were prepared from 1kg burdock.

The administration was given to each group once per day for 10 days in total. During the experimental process, the animals were observed everyday for their diets, survivals and behaviors and weighted every day. At the end of the experiment, the animals were anaesthetized and anatomize to take blood from abdominal vena cava for blood routine examination, including total white blood cell count and total platelet count.

### 3. Results

**Table 3: Effects of Arctigenin on white blood cell and platelet of mice received ⁶⁰Co radiation at 4Gy**

| Groups | Sample number | White Blood Cells (10⁹/L) | platelet (10⁹/L) |
|---|---|---|---|
| Group N | 10 | 4.926±0.50 | 678.2±78.5 |
| Group M | 10 | 2.535±0.38 | 482.0±54.2 |
| Group E | 10 | 3.242±0.45 | 524.5±74.6 |
| Group A | 10 | 4.225±0.51^{##$} | 596.2±72.5^{#$} |

| | | | |
|---|---|---|---|
| Compared with Group M: ^{#}P < 0.05, ^{##}P < 0.01; Compared with Group E: ^{$}P < 0.05. | | | |

As can be seen from the experimental results of the effects of Arctigenin on white blood cell and platelet of mice received ⁶⁰Co radiation at 4Gy (Table 3), Arctigenin exhibited unexpected effects in alleviating myelosuppression caused by radiotherapy. Specifically, the Arctigenin group shows an increase in both total white blood cell count and the platelet count with extremely significant difference (P < 0.01) or significant difference (P < 0.05) in comparison with the model group; and also a significant difference (*P* < 0.05) as comparing with burdock extract group.

### Example 13: Effects of Arctigenin on white blood cell and platelet of NOD mouse

Non-obese diabetic or NOD mice is a large mouse strain, including NOD/Scid, NOD/Ltj mice and the like. Yi Feng (Yi Feng, et al., "In vitro Expansion of Cord Blood Megakaryocyte Progenitor", ACTA Academiae Medicinae Sinicae, Vol.27, NO.2, pp 199-204) directly expanded megakaryocyte progenitor from cord blood CD34+ cells by using thrombopoietin (TPO), interleukin-11 (IL-11) and heparin in combination. Intravenous infusion of the megakaryocytes expanded for 7 days (at a concentration of 5 x 10⁶) into NOD/SCID mice received pre-treatment of radiation could accelerate the recovery of platelet and white blood cell account and improved the survival of the mice. The NOD/LTJ mice used in this example are immune-defect mice with significantly decreased white blood cell and platelet in comparison with normal mice.

### 1. Materials

1.1 Animals: 52 NOD/Ltj mice (purchased from Vital River Laboratory Animal Technology Co., Ltd., Beijing; Laboratory Animal NO: SCXK (BJ) 2006-2009), female, Age: 5 weeks, Body weight: 16-20 g.

### 1.2 Agents

Interleukin, purchased from Sino Biological Inc., Beijing;
Pentobarbital sodium, purchased from Shanghai Kefeng Chemical Reagent Co., Ltd.; Arctigenin, prepared according to Example 1; Purity 99.3%.

### 2. Groups and Administration

After one week of adaptive feeding, healthy NOD mice aged of 5 weeks were divided randomly into 4 groups:
I. Model Control Group: 13 animals;
II. Positive Control Group: 13 animals receiving gavage of interleukin at dose of 10.0 mg/kg every day;
III. Low-dose Arctigenin Group: 13 animals receiving gavage of interleukin at dose of 30 mg/kg every day;
IV. High-dose Arctigenin Group: 13 animals receiving gavage of interleukin at dose of 60 mg/kg every day.

The administration was given consecutively to each group once per day, and stopped after 15 days. The animals were then anaesthetized (3% pentobarbital sodium via intraperitoneal injection at 0.1~0.15 ml/animal) and 1 ml blood was taken via abdominal vena cava for blood routine examination.

**Table 4: Effects of Arctigenin on white blood cell and platelet of NOD mouse**

| Groups | Sample number | White Blood Cells (10⁹/L) | platelets (10⁹/L) |
|---|---|---|---|
| Model Control Group | 13 | 2.535±0.38 | 508.3±38.6 |
| Interleukin Group | 13 | 2.683±0.35 | 524.5±74.6 |
| Low-dose Arctigenin Group | 13 | 3.242±0.45^{#$} | 730.5±34.6^{##$} |
| High-dose Arctigenin Group | 13 | 4.225±0.51^{##$} | 780.2±46.5^{##$$} |

| | | | |
|---|---|---|---|
| Compared with Model Control Group: ^{#}P < 0.05, ^{##}P < 0.01; Compared with Interleukin Group: ^{$}P < 0.05, ^{$$}P < 0.01. | | | |

As can be seen from Table 4, the white blood cell counts and platelet counts of low-dose and high-dose arctigenin groups were significantly higher than these of model group, indicating significantly therapeutic effects. Compared with positive control (interleukin for preventing decrease of platelet) group, arctigenin groups exhibited significant advantage regarding increase of blood cell count (P < 0.05), and significantly increased platelet count, improved immunity. Especially, the high-dose arctigenin group showed significant advantage in its ability of increasing white blood cell count and platelet count, compared with the other groups.

The present invention provides a novel route for developing arctigenin, provides an ideal natural reagent for reducing toxic and side effects of radiotherapy or chemotherapy, and thus could bring favorably economic and social benefit.

## Claims

1. Arctigenin for use in preventing or treating bone marrow suppression induced by radiation or a chemical.

2. The arctigenin for use according to claim 1, wherein said chemical is a chemotherapeutic agent for treating cancer.

3. The arctigenin for use according to claim 2 wherein said chemotherapeutic agent is selected from the group consisting of docetaxel and imatinib mesylate.

4. The arctigenin for use according to any of claims 1 to 3, wherein said bone marrow suppression is **characterized in** decline in the number of peripheral blood leukocytes or thrombocytopenia, bone marrow dysplasia or aplastic anemia.

5. The arctigenin for use according to claim 4, wherein said peripheral leucopenia is **characterized in** reduction in peripheral blood neutrophils.

6. The arctigenin for use according to any one of claims 1 to 5, wherein arctigenin is used as the sole active ingredient.

7. A formulation comprising arctigenin and a pharmaceutically acceptable excipient for use in preventing or treating bone marrow suppression induced by radiation or a chemical, wherein the formulation is a microemulsion formulation having a mean particle diameter of 15-80nm.

## Patentansprüche

1. Arctigenin zur Anwendung bei der Prophylaxe oder Behandlung von durch Strahlung oder eine Chemikalie induzierter Myelosuppression.

2. Arctigenin zur Anwendung nach Anspruch 1, wobei es sich bei der Chemikalie um ein Chemotherapeutikum zur Behandlung von Krebs handelt.

3. Arctigenin zur Anwendung nach Anspruch 2, wobei das Chemotherapeutikum ausgewählt ist aus der Gruppe bestehend aus Docetaxel und Imatinibmesylat.

4. Arctigenin zur Anwendung nach einem der Ansprüche 1 bis 3, wobei die Knochenmarksuppression durch eine Abnahme der Anzahl an Leukozyten des peripheren Bluts oder Thrombozytopenie, Knochenmarkdysplasie oder aplastische Anämie gekennzeichnet ist.

5. Arctigenin zur Anwendung nach Anspruch 4, wobei die periphere Leukopenie durch eine Verringerung an Neutrophilen des peripheren Bluts gekennzeichnet ist.

6. Arctigenin zur Anwendung nach einem der Ansprüche 1 bis 5, wobei Arctigenin als einziger Wirkstoff angewandt wird.

7. Formulierung, umfassend Arctigenin und einen pharmazeutisch verträglichen Vehikels zur Verwendung bei der Prophylaxe oder Behandlung von durch Strahlung oder eine Chemikalie induzierter Myelosuppression, wobei es sich bei der Formulierung um eine Mikroemulsionsformulierung mit einem mittleren Teilchendurchmesser von 15 - 80 nm handelt.

## Revendications

1. Arctigénine pour une utilisation dans la prévention ou le traitement de la suppression de la moelle osseuse induite par rayonnement ou une substance chimique.

2. Arctigénine pour une utilisation selon la revendication 1, dans laquelle ladite substance chimique est un agent chimiothérapeutique pour traiter le cancer.

3. Arctigénine pour une utilisation selon la revendication 2, dans laquelle ledit agent chimiothérapeutique est choisi dans le groupe comprenant le docétaxel et le mésylate d'imatinib.

4. Arctigénine pour une utilisation selon l'une quelconque des revendications 1 à 3, dans laquelle ladite suppression de la moelle osseuse est **caractérisée par** une baisse du nombre de leucocytes du sang périphérique ou une thrombocytopénie, une dysplasie de la moelle osseuse ou une anémie aplasique.

5. Arctigénine pour une utilisation selon la revendication 4, dans laquelle ladite leucopénie périphérique est **caractérisée par** la réduction des neutrophiles du sang périphérique.

6. Arctigénine pour une utilisation selon l'une quelconque des revendications 1 à 5, dans laquelle l'arctigénine est utilisée comme unique ingrédient actif.

7. Formulation comprenant de l'arctigénine et un excipient pharmaceutiquement acceptable pour une utilisation dans la prévention ou le traitement de la suppression de la moelle osseuse induite par rayonnement ou une substance chimique, dans laquelle la formulation est une formulation de microémulsion ayant un diamètre moyen de particule de 15 à 80 nm.
